# EUROPEAN PATENT APPLICATION

(11) **EP 1 616 949 A1**
(43) Date of publication of application: **18.01.2006**
(21) Application number: 04726318.1
(22) Date of filing: 07.04.2004
(51) Int. Cl.: C12N 15/09, C12N 5/14, C12N 15/82, A01H 5/00

(54) **NOVEL VECTOR AND METHOD OF CONSTRUCTING TRANSFORMANT PLANT USING THE VECTOR**

(30) Priority: 07.04.2003 JP 2003135141
(71) Applicant: NIPPON PAPER INDUSTRIES CO., LTD., Kita-ku, Tokyo 114-0002 (JP)
(72) Inventor: KAWAOKA, Akiyoshi, Nippon Paper Industries Co.Ltd., Kita-ku, Tokyo 114-0002 (JP); NANTO, Kazuya, Nippon Paper Industries Co., Ltd., Kita-ku, Tokyo 114-0002 (JP); EBINUMA, Hiroyasu, Nippon Paper Industries Co.,Ltd, Kita-ku, Tokyo 114-0002 (JP)
(74) Representative: Ward, David Ian
(86) International application number: PCT/JP2004/005027
(87) International publication number: WO 2004/090130

(57) **Abstract**

Gene introduction to a plant is carried out by using a vector which comprises the following constitutional units A and B:
A: a DNA sequence comprising P1, an expression inhibitory sequence and a desired gene which is controlled by P1 and the expression inhibitory sequence;
B: a DNA sequence comprising P2, an expression inhibitory gene which is controlled by P2, P3, the expression inhibitory sequence, and a gene of a removal reaction-catalyzing enzyme which is controlled by P3 and the expression inhibitory sequence, wherein the DNA sequence is removed by expression of the gene of a removal reaction-catalyzing enzyme,

wherein P1 is a promoter which shows its activity in at least a callus and a plant tissue where a desired gene should be expressed, P2 is a promoter which shows its activity in at least a callus, and P3 is a promoter which shows its activity in at least a plant tissue where P1 and P2 do not show their activities.

## Description

### Technical Field

The present invention relates to a novel vector, and a method for producing a transgenic plant using the vector.

### Background Art

In recent years, some of the recombinant plants so far developed reached practical steps, and there are cases in which the produced recombinant plants are actually grown in the field. However, in this case, since there is a possibility of causing crossing of the recombinant gene-containing pollen with wild species, scattering of the pollen becomes a problem. Accordingly, in order to prevent this problem, studies are in progress on the techniques for inhibiting pollen formation of plants, namely sterility techniques, at the same time with the development of such recombinant plants. Also, since pollinosis by cedar pollen and the like became a social problem in recent years, such studies on the sterility techniques are also in progress for the prevention of pollinosis in the case of a plant such as a cedar. In addition, regarding the floriculture, studies on the sterility are in progress also for the purpose of improving flowering, prolonging flowering period and the like, by preventing lowering of vegetation accompanied by seed formation.

Thus, social demand for the study on sterile plants is very large. In the past, introduction of sterile character into plants have been carried out mainly using crossing and genetic engineering techniques. Introduction of sterile character by crossing has merits in that special devices and facilities are not necessary and it can be carried out by convenient means, but on the other hand, it has a demerit in that applicable plant species are limited. That is, currently, agricultural plants in which individuals having sterile character are confirmed are rice plant, wheat, corn, rapeseed and the like, but a sterile plant cannot be prepared when the plant is not crossable with these plants.

On the other hand, introduction of sterile character by genetic engineering techniques has a merit of being applicable to a broad range of plant species unlike the case of the method by crossing. Up to now, corn, cabbage, broccoli, lettuce, melon and the like are known as the plants which were successful in obtaining sterility by genetic engineering techniques, and for example, there is a report in International Publication WO89/10396 stating that a male sterile gene was prepared by connecting a Barnase gene to the downstream of expression promoter specific for the tapetum cell of tobacco anther tissue, and was introduced into the plant to obtain a sterile plant.

However, even in the case of a so-called tissue- or organ-specific promoter (hereinafter simply referred to as tissue-specific promoter), almost all of the promoters which function in plant cells are activated in calluses. This is considered that, since callus is a mass of dedifferentiated cells in which meristematic activity of the cells is high and deviated from the tissue-specific expression control, the transcriptional regulatory factor which takes charge of the tissue or organ specificity does not function. However, in preparing a sterile plant by introducing a sterile gene into a plant, it generally mediates callus in the process. In most cases of the genes to be used in sterilization, including the above-described Barnase gene, a gene capable of inhibiting cell functions in certain forms is formed by being positioned under regulation of promoters which specifically take actions in floral meristematic cells, and these promoters are also activated not only in floral meristematic cells and anthers but also in calluses. Thus, there was a phenomenon in which an intended sterile plant cannot be obtained because of the death of cells and obstruction of their growth due to expression of a cell function inhibitory gene in callus cells, during the process of preparing a sterile plant from plant cells after gene introduction treatment.

The above-described expression of a desired gene in callus cells in the process of recombinant plant production has undesirable influences such as inhibition of the redifferentiation of adventitious buds and the like, not only in a case where a cell function inhibitory gene for preparing a sterile plant is used, as a desired gene, together with a floral meristematic cell-specific promoter, but also in a case where other useful gene is used together with a tissue-specific promoter. In fact, there are many cases which are considered to be a failure in producing recombinant plants caused by this influence.

Taking the above problems into consideration, the present invention has been carried out in order to provide a technique for properly controlling expression of a desired gene according to the culturing stage and the culture tissue or organ when the desired gene is introduced into a plant cell by genetic engineering techniques, followed by culturing to produce a recombinant plant.

Particularly, an object of the present invention is to provide a technique for inhibiting expression of the desired gene in calluses which sometimes results in a failure in producing recombinant plants.

In addition, another object of the present invention is to provide a technique which produces a large effect in preparing sterile plants.

### Disclosure of the Invention

The invention relates to the following (1) to (11).
(1) A vector which comprises the following constitutional units A and B positioned on the same DNA molecule or on different DNA molecules, respectively, said vector comprising:
   a promoter, as Promoter 1, which shows its activity in at least a callus and a plant tissue where a desired gene should be expressed,
   a promoter, as Promoter 2, which shows its activity in at least a callus, and
   a promoter, as Promoter 3, which shows its activity in at least a plant tissue where Promoter 1 and Promoter 2 do not show their activities,
      A: a DNA sequence comprising Promoter 1, an expression inhibitory sequence and a desired gene in which expression is accelerated by Promoter 1 and expression is inhibited by the expression inhibitory sequence;
      B: a DNA sequence comprising Promoter 2, an expression inhibitory gene in which expression is accelerated by Promoter 2 to exert function of expression inhibition by the expression inhibitory sequence, Promoter 3, the expression inhibitory sequence, and a gene of a removal reaction-catalyzing enzyme in which expression is accelerated by Promoter 3 and expression is inhibited by the expression inhibitory sequence, wherein the DNA sequence is removed by expression of the gene of a removal reaction-catalyzing enzyme.
(2) The vector according to the above-described (1), wherein
   Promoter 1 is a promoter which shows its activity in at least a floral meristematic cell and a callus, but does not show its activity in a meristematic cell of a bud;
   Promoter 2 is a promoter which shows its activity in at least a callus, but does not show its activity in a meristematic cell of a bud, Promoter 3 is a promoter which shows its activity in at least a meristematic cell of a bud, and
   the desired gene is a cell function inhibitory gene.
(3) The vector according to the above-described (1) or (2), wherein Promoter 1 and Promoter 2 each is a promoter which regulates expression of PISTILLATA (PI) gene, APETALA1 (AP1) gene, APETALA2 (AP2) gene, APETALA3 (AP3) gene, AGAMOUS (AG) gene, LEAFY (LFY) gene and/or SEPALLATA3 (SEP3) gene of *Arabidopsis thaliana,* or a tobacco TA29 promoter.
(4) The vector according to any one of the above-described (1) to (3), wherein Promoter 3 is a histone H3 promoter or a histone H4 promoter of a plant, or a promoter which regulates expression of SHOOT MERISTEMLESS (STM) gene and/or CUP-SHAPED COTYLEDOM (CUC) gene *of Arabidopsis thaliana.*
(5) The vector according to any one of the above-described (1) to (4), wherein the desired gene is a gene encoding a cytotoxin.
(6) The vector according to the above-described (5), wherein the gene encoding a cytotoxin is a gene encoding Bax, RNase, a protease or a DAM methylase.
(7) The vector according to any one of the above-described (1) to (6), wherein the expression inhibitory sequence is an operator sequence and the expression inhibitory gene is a gene encoding an operator sequence-binding protein.
(8) The vector according to the above-described (7), wherein the operator sequence is a sequence in which a transcription activation region is deleted from lacI gene or yeast GAL4 gene.
(9) The vector according to any one of the above-described (1) to (8), wherein the gene of a removal reaction-catalyzing enzyme is a recombinant enzyme gene in a site-specific recombination system, and the constitutional unit B is interposed between two recognizing sequences which are recognized by a the recombinant enzyme encoded by the recombinant enzyme gene and mutually face the same direction.
(10) A method for producing a plant transformant while inhibiting expression of a desired gene in a callus, which comprises introducing a gene into a plant cell using the vector according to the above-described (1), and culturing the plant cell to redifferentiate a plant tissue or organ via a callus.
(11) A method for producing a sterile plant, which comprises introducing a gene into a plant cell using the vector according to any one of the above-described (2) to (9), culturing the plant cell to propagate a callus, and redifferentiating a bud from the callus to produce a plant individual.

### Brief Description of the Drawings

Fig. 1 is a drawing showing basic constitution of the vector of the present invention.
Fig. 2 is a drawing showing a restriction enzyme map of pMS-Bax1.
Fig. 3 is a photograph showing conditions of dead flower bud tissue observed in the tobacco plant obtained in Example 1.
Fig. 4 is a photograph showing a result of PCR for a genomic DNA extracted from a tobacco leaf in which flower bud tissue was perished in Example 1.
Fig. 5 is a photograph showing a result of Northern hybridization of a callus (C) obtained in the process of producing a tobacco plant of a system in which flower bud tissue was perished and RNA (S) extracted from an adventitious bud redifferentiated thereafter.
Fig. 6 is a drawing showing the function of pMS-Bax1.
Fig. 7 is a drawing showing a restriction enzyme map of pMS-Bax1-ipt.

### Best Mode for Carrying Out the Invention

The present invention is described below in detail.

### (1) Vector of the present invention

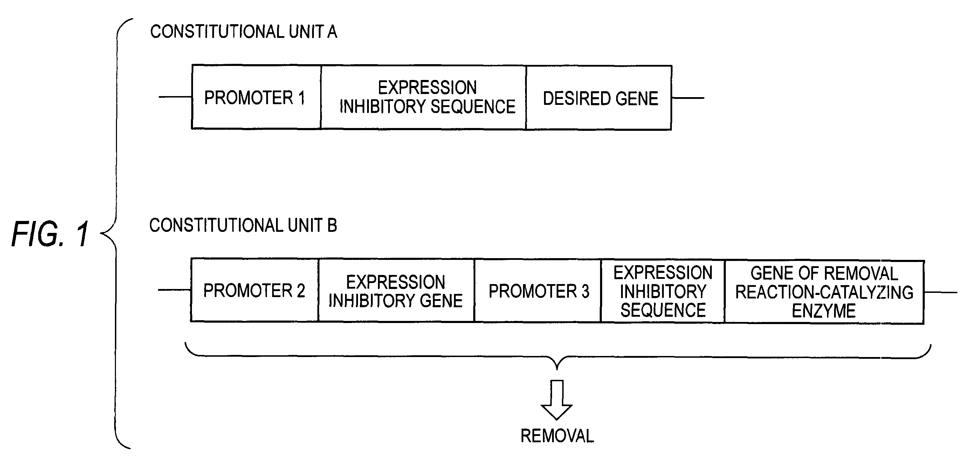

As shown in Fig. 1, the vector of the present invention is roughly constructed by two constitutional units. One is a constitutional unit A which comprises a DNA sequence comprising Promoter 1, an expression inhibitory sequence and a desired gene in which expression is accelerated by Promoter 1 and expression is inhibited by the expression inhibitory sequence. The other one is a constitutional unit B which comprises a DNA sequence comprising Promoter 2, an expression inhibitory gene in which expression is accelerated by Promoter 2 to exert function of expression inhibition by the expression inhibitory sequence, Promoter 3, the expression inhibitory sequence, and a gene of a removal reaction-catalyzing enzyme in which expression is accelerated by Promoter 3 and expression is inhibited by the expression inhibitory sequence, wherein the DNA sequence is removed by expression of the gene of a removal reaction-catalyzing enzyme.

Also, the vector according to the present invention is not always limited to one consisting of one DNA molecule, and it also means one consisting of two DNA molecules. Accordingly, the above-described constitutional units A and B are not necessarily present on the same DNA molecule and may be positioned on respectively different DNA molecules.

Next, each of the above-described constitutional elements is described.

As Promoter 1, a promoter which shows its activity in at least a callus and a plant tissue where a desired gene should be expressed to thereby accelerate expression of the desired gene under its control is used. As described above, most of the promoters which function in plant cells, including those which are called tissue-specific promoters, show their activities in calluses. Accordingly, as Promoter 1, on the basis of a plant tissue in which the desired gene is expressed under this control, a promoter capable of showing its activity in this plant tissue may be selected and used.

Also, as Promoter 2, a promoter which shows its activity in at least a callus to thereby accelerate expression of a gene under its control is used. In addition, as Promoter 3, a promoter capable of showing its activity in a plant tissue in which Promoter 1 and Promoter 2 do not show their activities is used.

Also, in such a vector, a vector useful for the production of a sterile plant can be obtained by using, as Promoter 1, a promoter which shows its activity in a floral meristematic cell, but does not show its activity in a meristematic cell of a bud; as Promoter 2, a promoter which shows its activity in at least a callus, but does not show its activity in a meristematic cell of a bud; and as Promoter 3, a promoter which shows its activity at least in a meristematic cell of a bud and also using a cell function inhibitory gene as the desired gene under the control of Promoter 1.

In this case, examples which can be used as Promoter 1, namely a promoter which shows its activity in a floral meristematic cell and a callus, but does not show its activity in a meristematic cell of a bud, include promoters which control expression of PISTILLATA (PI) gene, APETALA1 (AP1) gene, APETALA2 (AP2) gene, APETALA3 (AP3) gene, AGAMOUS (AG) gene, LEAFY (LFY) gene and/or SEPALLATA3 (SEP3) gene of *Arabidopsis thaliana* which functions in floral meristematic cells, a tobacco TA29 promoter which specifically functions in the anther among floral meristematic cells, and the like. In addition, all of these can also be used as Promoter 2.

Also, examples which can be used as Promoter 3, namely a promoter which shows its activity in meristematic cells of buds, include promoters capable of controlling expression of a histone H3 promoter and a histone H4 promoter of a plant, SHOOT MERISTEMLESS (STM) gene and/or CUP-SHAPED COTYLEDOM (CUC) gene *of Arabidopsis thaliana,* and the like.

In addition, regarding the cell function inhibitory gene, its kinds are not limited, so long as it is a gene encoding a protein which damages function of cells, and examples which can be used include genes encoding RNase, a protease, a DAM methylase and a cytotoxin such as diphtheria toxin, which are conventionally used in producing sterile plants, can be used. Also, Bax gene was used as the cell function inhibitory gene in Examples of the present invention, and this is a gene encoding a mammal Bax which induces apoptosis.

In the vector of the present invention, any combination of the expression inhibitory sequence with the expression inhibitory gene which exerts function of expression inhibition by the expression inhibitory sequence can be used. Typically, an operator sequence can be used as the expression inhibitory sequence, and a gene encoding an operator sequence-binding protein can be used as the expression inhibitory gene. However, in addition to this combination, for example, it may be a combination of an expression inhibitory sequence having a predetermined nucleotide sequence with an expression inhibitory gene which becomes the template of mRNA having antisense relation with this sequence. In this case, the expression inhibitory gene inhibits expression of a gene positioned in the downstream of this expression inhibitory sequence, by blocking expression from the expression inhibitory sequence with mRNA having antisense relation with this. That is, at this time, the expression inhibitory gene exerts function of expression inhibition by the expression inhibitory sequence. In addition, such an expression inhibition by the expression inhibitory gene and expression inhibitory sequence can also be carried out by utilizing RNA interference.

Also, it is preferable to use a lactose operon derived from *Escherichia coli* as the gene encoding an operator sequence-binding protein, and a DNA sequence capable of recognizing this lactose operon, as the operator sequence. In the case of a gene derived from a plant, an endogenous paralog gene may have influence upon the expression control of the desired gene. In addition, in order to bind the operator sequence-binding protein to the operator sequence more effectively, 2 or more of this operator sequence may be positioned in a row.

The gene of a removal reaction-catalyzing enzyme and the mechanism for removing the above-described constitutional unit B by the expression of this gene of a removal reaction-catalyzing enzyme are already conventionally known by those skilled in the art, and for example, a site-specific recombination system and a transposon can be used as described in Japanese Patent 3256952. Also, in Examples of the present invention, by utilizing the site-specific recombination system, the constitutional unit B is removed by using a recombinant enzyme gene in the site-specific recombination system as the gene of a removal reaction-catalyzing enzyme and by interposing the whole constitutional unit B between two recognizing sequences which are recognized by this recombinant enzyme gene and are mutually facing the same direction.

Also, the site-specific recombination system is a system in which removal or recombination of the region interposed between recognition sequences is induced by the actions of a recombinase and the recognition sequences upon which this recombinase acts by recognizing them. That is, when two of this recognition sequence are present on the same DNA molecule facing the same direction at a certain interval, the region interposed by them is removed from this DNA molecule, and when two of this sequence are present facing opposite directions, this region turns around, so that the former removal action is used in the present invention.

Up to now, a Cre/lox system, an R/RS system, an FLP system, a cer system, a fim system and the like separated from microorganisms such as phage, bacteria (e.g., *E. coli)* and yeast are known as the site-specific recombination system (as an introduction, N.L. Craig, *Annu. Rev. Genet., 22:* 17 (1988)), but its presence is not known yet in plants and other higher organisms. However, it has been found that when these site-specific recombination systems separated from microorganisms are introduced into an organism species (including plants) different from the original organism species, they show the same behavior as the behavior in the original organisms. Among these, the R/RS system (H. Matsuzaki *et al., J. Bacteriology, 172:* 610 (1990)) which is a site-specific recombination system of a yeast *(Zygosaccharomyces rouxii)* has high removal efficiency and therefore is preferred as the site-specific recombination system to be used in the present invention.

According to the vector of the present invention, in addition to these composing elements, a selectable marker gene, a DNA sequence encoding a DNA binding region, a DNA sequence encoding an inactivation domain and the like may be further positioned.

As the selectable marker gene, generally used selectable marker genes such as an antibiotic resistance gene and a herbicide resistance gene can be used. For example, one or more genes selected from a kanamycin resistance gene (NPTII), a hygromycin phosphotransferase (htp) gene which provides plants with resistance for an antibiotic hygromycin, a phosphino-thricin acetyl transferase (bar) gene which provides resistance for bialaphos and the like can be used. Insertion of such a selectable marker gene into the vector of the present invention is effective for the efficient selection of a desired transformed cell.

Particularly, when a plant hormone synthesis gene, such as an isopentenyl transferase (ipt) gene encoding an enzyme which synthesizes cytokinin or an indoleacetamide hydroxylase (iaaH) gene encoding an enzyme which synthesizes auxin, or a gene concerned in the signal transduction of plant hormones, such as msh1 or cki1, is introduced as a negative selectable marker gene into the constitutional unit B, the presence or absence of the removal of this constitutional unit B can be detected with the naked eye as the presence or absence of the generation of malformation of a plant tissue or organ, so that the method of the present invention can be efficiently carried out.

Also, it is known in general that an introduced gene is introduced into various positions in the genome of a host plant and shows different expression depending on the introduced positions, and this is called position effect. When a constantly expressing appropriate promoter, a reporter gene such as a gene encoding β-glucuronidase (GUS) or luciferase (LUC) are inserted into the vector of the present invention, a transformant which effectively expresses the constitutional elements of the vector of the present invention such as the desired gene can be easily selected by this position effect. Also, the transformant according to the present invention means the whole plant individual, plant organ or plant tissue into which the desired gene is introduced by the vector of the present invention.

In addition, in order to express a foreign gene in a plant individual, it is necessary to position a terminator for plant or the like in the downstream of the structural gene. Accordingly, when simply called gene in this specification, it means the structural gene and its terminator sequence. Also in the case of the respective gene which forms the above-described constitutional units A and B, it has a terminator sequence in the downstream of its structural gene. Also, examples of the terminator sequence include a cauliflower mosaic virus-derived, a nopaline synthase gene-derived and the like. However, any terminator can be used in the present invention without limiting thereto, so long as the terminator is known to function in the plant individual.

### (2) Introduction of the vector of the present invention into plant host

The vector of the present invention can be introduced into cells of a host plant using conventionally known gene introduction methods. Typical gene introduction methods include indirect introduction methods such as an agrobacterium infection method, and direct introduction methods such as a particle gun method, a polyethylene glycol method, a liposome method and a microinjection method. For example, when the agrobacterium infection method is used, the vector of the present invention may be introduced as follows.

Firstly, a recombinant vector for use in the gene introduction into plants is obtained by inserting the vector of the present invention into a cloning vector for plant cell in the usual way. In this case, binary vector system plasmids such as pBI2113Not, pBI2113, pBI101, pBI121, pGA482, pGAH and pBIG, and intermediary vector system plasmids such as pLGV23Neo, pNCAT and pMON200 can be used as the vector for cloning.

For example, when a binary vector system plasmid is used, the constitutional unit A and/or B of the vector of the present invention is inserted into a T-DNA region interposed between boundary sequences LB and RB in the above-described binary vector, and this recombinant vector is amplified in *E. coli.* Next, the thus amplified vector is introduced into *Agrobacterium tumefaciens* (hereinafter simply referred to as *"A. tumefaciens")* C58, LBA4404, EHA101, C58C1RifR, EHA105 or the like by a freeze-thawing method, an electroporation method, a ternary conjugation method *(Nucleic Acids Research,* 12: 8711 (1984)) or the like, and a plant cell is infected with the *Agrobacterium* to thereby introduce the vector of the present invention into the plant.

Also, when a binary vector system plasmid is used, the constitutional unit A and B constructing the vector of the present invention is preferably positioned in the same T-DNA region, but it may be positioned in different T-DNA regions. Also, they may be respectably inserted into different plasmids.

In the present invention, kinds of the plant to be used as the host are not limited. For example, *Arabidopsis thaliana,* tobacco, rice, corn, poplar, eucalyptus, cedar or the like can be used as the host. The above-described gene introduction method can be carried out by using any one of cultured cells, the whole plant individuals, organs (e.g., leaf, petal, stem, root, rhizome, seed, *etc.)* or tissues (e.g., epidermis, phloem, parenchyma, xylem, vascular bundle, *etc*.) of each of these plants. In addition, when the electroporation method is used, it can be carried out using protoplasts as the object.

### (3) Production of transformant

The plant cell after the gene introduction treatment in the present invention is firstly cultured using an appropriate callus induction medium to propagate a callus. As a result, the expression inhibitory gene is expressed and exert expression inhibition action by the expression inhibitory sequence, by the action of Promoter 2 which shows its activity in calluses, and expression of the desired gene and gene of a removal reaction-catalyzing enzyme is inhibited. Thus, the desired gene is not expressed and the constitutional unit B is not removed in the callus.

Next, this callus is transplanted to an appropriate redifferentiation medium to redifferentiate a plant tissue or organ in which Promoter 3 shows its action. Since Promoter 1 and Promoter 2 do not show their activities in such a tissue or organ, the desired gene is not expressed, and expression of the gene of a removal reaction-catalyzing enzyme is not prevented by the expression inhibitory sequence. Accordingly, the gene of a removal reaction-catalyzing enzyme is expressed, and the constitutional unit B is removed.

Thereafter, the transformant of interest is obtained by redifferentiating a tissue or organ in which the desired gene is expressed. In this case, since the constitutional unit B was already removed, expression of the desired gene is not inhibited by the expression inhibitory sequence.

That is, in the vector of the present invention, expression of the desired gene is controlled under a promoter which acts in a cascade-like manner and a regulatory factor comprising the expression inhibitory gene and the gene of a removal reaction-catalyzing enzyme. Therefore, when the desired gene is introduced into a plant cell using the vector of the present invention, expression of the desired gene is appropriately controlled according to the culturing stage or according to the cultured tissue or organ. Accordingly, by simply culturing the cell after gene introduction treatment and redifferentiating a plant tissue or organ from this cell via a callus, a transformant in which this desired gene is expressed in a specified tissue or organ can be produced.

Particularly, in the case where Promoter 1 is a promoter which shows its activity in at least a floral meristematic cell and a callus, but does not show its activity in a meristematic cell of a bud, Promoter 2 is a promoter which shows its activity in at least a callus, but does not show its activity in a meristematic cell of a bud, Promoter 3 is a promoter which shows its activity in at least a meristematic cell of a bud, and the dersired gene is a cell function inhibitory gene, when a plant cell after the gene introduction treatment is cultured to propagate a callus and then a bud is redifferentiated from this callus to redifferentiate a flower bud, the cell function inhibitory gene is not expressed during growth of the callus but is expressed at the time of the commencement of the flower bud redifferentiation and inhibits the flower bud formation to cause death of dividing cells of the flower bud, so that a sterile plant can be obtained. In order to obtain a flower bud, namely primordium of a flower bud to be exact, from a redifferentiated bud, this bud is elongated and cut off, a young plant individual is regenerated by planting it in an appropriate rooting medium for rooting, and this young plant individual is grown to induce redifferentiation of a flower bud.

Also, the callus inducing medium for use in the growth of a callus from a plant cell and the redifferentiation medium for use in the redifferentiation of a plant tissue or organ from a callus can be prepared by optionally adding a plant hormone, such as cytokinin or auxin, and the like to a medium conventionally known as a plant tissue culture medium. In addition, when an antibiotic resistance gene or an agricultural chemical resistance gene as described above is used as a selectable marker gene, an antibiotic or an agricultural chemical corresponding to such a resistance gene may be added to the medium for the selection of a transformant.

### (4) Detection and confirmation of transformant

Detection of a transformant produced by the vector of the present invention and confirmation of stability in its after age can be carried out by extracting DNA from the cells or tissues thereof in the usual way and analyzing it by a conventionally known method such as PCR or Southern blotting method and the like.

In addition, when the desired gene to be introduced by the vector of the present invention has influence upon external appearances of the resulting plant individual, such as provision of a sterile character and the like, detection and confirmation of a transformant can also be carried out by finally regenerating a young plant individual from a plant cell after the gene introduction treatment, or further growing this young plant individual, and examining its external appearance. For example, when a sterile plant is prepared by the vector of the present invention, the transformant can be detected and confirmed by planting and growing a young plant individual obtained in the above-described manner in a flower pot filled with culture soil such as Metromix 350, and examining formation of a flower bud after a lapse of a predetermined period.

The present invention is described below with reference to examples; however, the present invention is not limited thereto.

### 1. Expression of PI promoter in tobacco plant

PISTILLATA (PI) is known as one of the promoters which show their activities in a floral meristematic cell and a callus, but do not show the activities in a meristematic cell of a bud (*Goto et al., Genes & Development, 8:* 1548-1560 (1994)). In this example, firstly, in order to examine whether or not the promoter shows the activity in a tobacco plant, similer to the activity in the case of *Arabidopsis thaliana,* a binary vector system recombinant plasmid having the PI promoter and a fusion gene of GUS gene, taken over from Dr. H. Goto at Institute for Chemical Research, Kyoto University (presently Okayama Prefectural University), was introduced into A. *tumefaciens* EHA105 by the electroporation method.

Next, under sterile conditions, a leaf of a tobacco plant (*Nicotiana tabacum* SR-1) grown from a seed for about 4 weeks was cut into a section of 5 mm square and soaked in a culture medium prepared by culturing the *A. tumefaciens* overnight at 28°C in the LB medium containing 50 mg/l of kanamycin, for about 1 to 3 minutes by placing it such that epidermis of the leaf became downward, and after removing the culture medium adhered to its surface with sterilized paper towel or the like, put onto a callus induction medium (MS inorganic salts, 3% sucrose, 0.8% agar, 1 mg/l 1-naphthaleneacetic acid, 0.1 mg/l benzyladenine) for co-culturing at 25°C under continuous illumination.

Three days after the co-culturing on the callus induction medium, the above-described section was transplanted to a medium for shoot formation (MS inorganic salts, 3% sucrose, 0.25% gellan gum, 0.1 mg/l 1-naphthaleneacetic acid, 1 mg/l benzyladenine, 100 mg/l kanamycin, 500 mg/l carbenicillin) to continue the culturing, the differentiated foliage about 4 weeks thereafter was cut off, and this foliage was transplanted into a culture bottle (40 φ x 130 mm) charged with the MS basal medium containing 100 mg/l kanamycin and 500 mg/l carbenicillin and cultured for about 4 weeks for rooting to thereby obtain a rooted individual of the transformed tobacco plant having PI promoter::GUS fusion gene.

The rooted individual obtained in this manner was further replanted into a pot charged with a vermiculite (manufactured by Nihon Taika Kogyo)/peat moss (Izumi Nozai) mixed soil and grown in an greenhouse of 25°C to collect flower buds, flower organs, shoot apex parts, stems, leave and roots, and the GUS activity of cells constituting these tissues was examined by the method of Jefferson *et al. (Plant Mol. Biol. Rep.,* 5: 387-405 (1987)). In addition, a callus was obtained from the rooted individual obtained in this manner, and the GUS activity of this callus was also examined. As a result, high GUS activity was detected in the floral meristematic cell, stamen and callus at the initial stage of flower bud formation, thus confirming that the above-described PI promoter shows its activity in these tissues.

### 2. Separation of cell function inhibitory gene

In order to use a human derived Bax gene as the cell function inhibitory gene, which has already been reported stating that the gene has action to damage the function of a mitochondria which takes charge of energy production of cells, and cells die out when the gene is expressed in a large quantity also in the case of plants (Laccomme *et al., PNAS,* 96: 7956-7961, 2000), the gene was separated by the PCR method.

That is, based on the sequence information of Bax gene obtained from DDBJ, an hBAX1 primer (AGGCCCCGGG GGGAGCGGCG GTGATGGCG; SEQ ID NO: 1) containing a recognition site of a restriction enzyme *Sma*I and an Hbax2 primer (CAGAGCTCTG CCATAATTTA TGGAGGAAAA, SEQ ID NO:2) containing a recognition site of a restriction enzyme *Sac*I were synthesized, and using these primers and using a human brain derived cDNA library (manufactured by Takara Bio) as the template, PCR was carried out and the reaction product was separated by an agarose gel electrophoresis. As a result, since amplification of a 650 bp DNA fragment considered to be the human Bax gene was detected, this DNA fragment was recovered, a portion thereof was separated, and its nucleotide sequence was examined using a sequencer (Beckman, CEQ2000XL) to confirm that it completely coincides with the human Bax gene. In this case, the PCR of this time was carried out using TaKaRa LA PCR Kit Ver2.1 (manufactured by Takara Bio).

### 3. Construction of constitutional unit A

A constitutional unit A was constructed using the above-described PI promoter as Promoter 1, an operator sequence, as the expression inhibitory sequence, which is recognized by an E. *coli* LacI gene product and binds thereto, and the above-described Bax gene as the desired gene.

Firstly, two kinds of oligo DNA, -GATCCATTGT GAGCGCTCAC AATACGTATT GTGAGCGCTC ACAAT- (SEQ ID NO:3) and -GATCATTGTG AGCGCTTAC AATACGTATTG TGAGCGCTCA CAATT- (SEQ ID NO:4), in which respective 5' side was phosphorylated, were synthesized and subjected to annealing treatment by gradually lowering from 94°C to room temperature, and by pairing them, a double-stranded DNA containing the operator sequence (hereinafter simply referred simply to as operator sequence) was obtained. In this double-stranded DNA, its 5'-terminal and 3'-terminal respectively form protruding region and excessive region which are recognized by a restriction enzyme *Bam*HI.

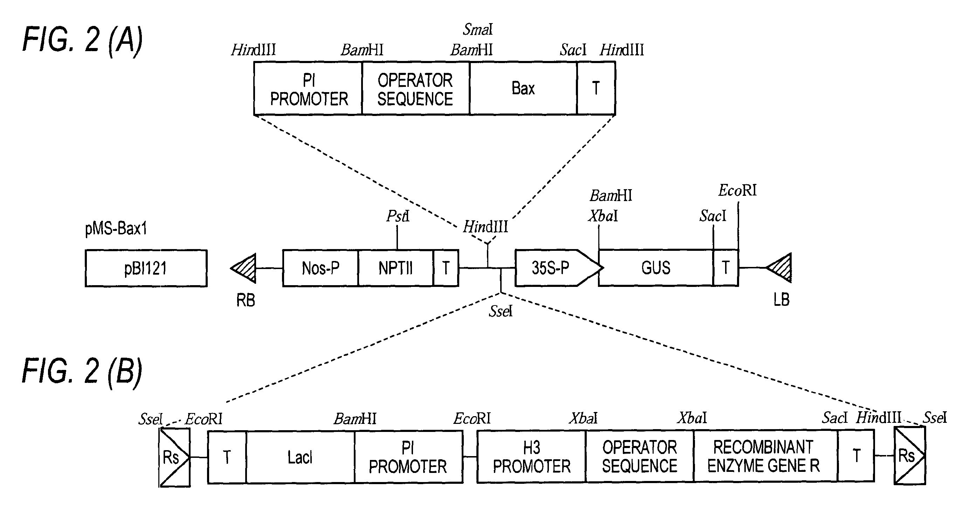

Next, the constitutional unit A was constructed by connecting this synthetic double-stranded DNA between PI promoter and Bax gene (Fig. 2 (A)). In this constitutional unit A, expression of Bax gene in calluses and floral meristematic cells is accelerated by the action of PI promoter, but expression of Bax gene is inhibited even in these cells when the LacI gene product is bound to the above-described operator sequence.

### 4. Construction of constitutional unit B

A constitutional unit B was constructed by connecting, to the PI promoter, the LacI gene as an expression inhibitory gene encoding a protein capable of recognizing the operator sequence prepared in the above-described 3 and of binding thereto, by connecting an operator sequence which is recognized by a LacI gene product and binds thereto and a gene of a recombinant enzyme R in this order to a eucalyptus derived histone H3 promoter which shows its activity in meristematic cells of buds and calluses, and by further inserting these composing elements into restriction enzymes *Hin*dIII and *Eco*RI recognition sites in a cassette interposed between a pair of the recognition sequence RS facing the same direction and upon which the recombinant enzyme R takes action by recognizing it (Fig. 2 (B)).

Also, the operator sequence connected between the above-described histone H3 promoter and recombinant enzyme R gene is a double-stranded DNA obtained by annealing two kinds of oligo DNA, -CTAGAATTGT GAGCGCTCAC AATACGTATT GTGAGCGCTC ACAAT- (SEQ ID NO:5) and -CTAGATTGTG AGCGCTCACA ATACGTATTG TGAGCGCTCA CAATT- (SEQ ID NO:6), and is different from the operator sequence prepared in the above-described 3 only from the viewpoint that its 5'-terminal and 3'-terminal respectively form protruding region and excessive region which are recognized by a restriction enzyme *Xba*I. However, this operator sequence also inhibits expression of a gene connected to its downstream and functions as an expression inhibitory sequence of the constitutional unit B, by the binding of the LacI gene product. In addition, the recombinant enzyme R and the recognition sequence RS are derived from a site-specific recombination system R/RS of a soy sauce yeast *(Zygosaccharomyces rouxii).*

In this constitutional unit B, expression of LacI gene in calluses and floral meristematic cells is accelerated by the action of PI promoter. On the other hand, the histone H3 promoter functions in calluses and meristematic cells of buds, but since the LacI gene product binds to the operator sequence connected thereto, expression of the R gene existing in its downstream is inhibited. However, in meristematic cells of buds, the LacI gene is not expressed because the PI promoter does not function therein, and the recombinant enzyme R is produced due to acceleration of expression of the R gene by the action of this histone G3 promoter and the cassette interposed between the recognition sequences Rs is removed.

### 5. Construction of vectorpMS-Bax1 for plant gene introduction

A vector pMS-Bax1 into which the vector of the present invention was introduced for use in the gene introduction into plants (this has been deposited on April 7, 2003, as FERM BP-8352 in International Patent Organism Depositary, National Institute of Advanced Industrial Science and Technology (Tsukuba Central 6, 1-1, Higashi 1-chome, Tsukuba-shi, Ibaraki 305-8566, JAPAN)) was obtained by connecting the thus constructed constitutional unit A (Fig. 2 (A)) and constitutional unit B (Fig. 2 (B)) and inserting between RB site and LB site of a vector pBI121 for plant gene introduction.

### 6. Introduction of pMS-Bax1 into tobacco plant

In the same manner as in the above-described 1, pMS-Bax1 was introduced into *A. tumefaciens* EHA105, subsequently, a tobacco plant *(Nicotiana tabacum* SR-1) was infected with this pMS-Bax1-introduced *A. tumefaciens,* a callus was induced from the infected tobacco plane, an adventitious bud was regenerated for rooting, and then the seedling was grown to thereby obtain a transformed tobacco plant into which the vector of the present invention was introduced.

### 7. Morphology of flower bud tissue

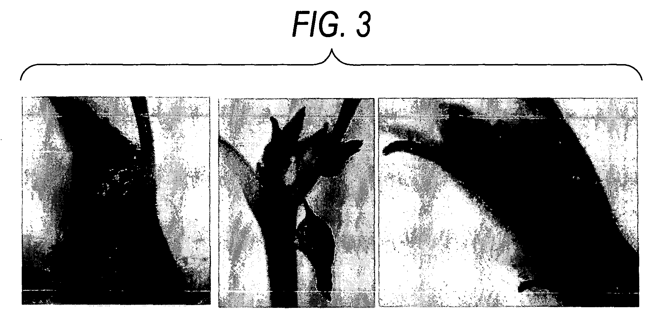

When morphology of flower buds of the transformed tobacco plant obtained in the above-described manner was observed with the naked eye, it was revealed that the flower bud tissue was died out in 5 individuals among 12 individuals (Fig. 3).

In addition, based on this observation result, the presence of recombinant enzyme R gene, recognition sequences Rs and Bax gene was examined on a leaf of a transformed tobacco plant individual considered to be sterilized because of the die out of flower bud tissue. That is, a genomic DNA was extracted from a leaf of this transformant in the usual way, PCR was carried out using the genomic DNA as the template and using specific primers which could recognize the above-described genes and recognition sequences, and the presence of these genes or recognition sequences was examined by analyzing the amplified products by electrophoresis.

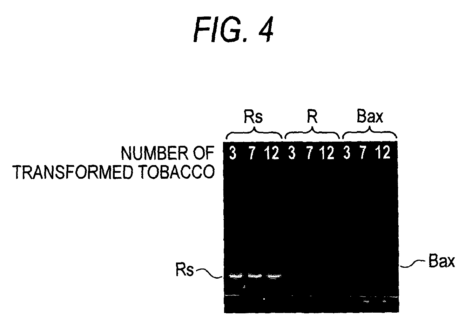

The results are shown in Fig. 4. As shown in Fig. 4, in the leaf of a transformed tobacco plant individual considered to be sterilized because of the die out of the flower bud tissue, amplified products of the recognition sequences Rs and Bax gene were detected so that their presence was confirmed, but no amplified product of the R gene was detected.

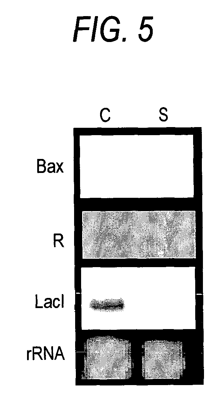

Also, Fig. 5 shows a result in which RNA was extracted from calluses and adventitious buds obtained during the process of producing such transformants and Northern blotting was carried out on these RNA samples. As shown in Fig, 5, it was able to detect expression of LacI gene and weak expression of R gene in a callus obtained just after the gene introduction treatment using the vector of the present invention, but expression of Bax gene was not detected. On the other hand, in the case of an adventitious bud redifferentiated from this callus, expression of LacI gene, R gene and Bax gene was not detected.

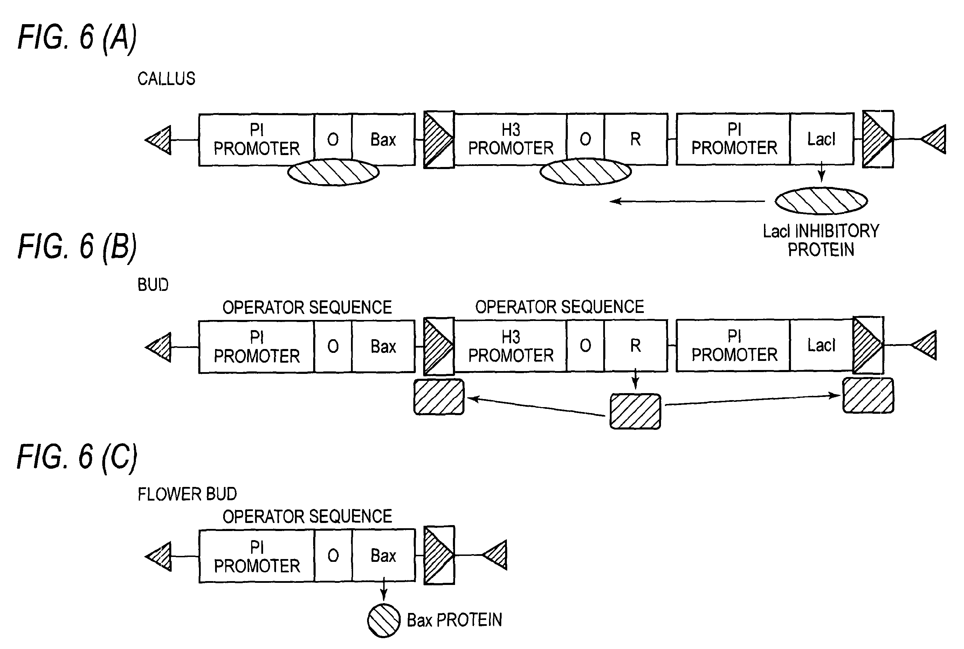

Based on the above, it is considered that this vector pMS-Bax1 functioned in the tobacco plant into which this vector has been introduced, in the manner as shown in Fig. 6. That is, in the callus obtained just after the gene introduction treatment, LacI protein is produced trough the expression of the expression inhibitory gene LacI induced by the action of PI promoter, and this binds to the operator sequence to cause inhibition of the expression of Bax gene and R gene (Fig. 6(A)). However, when an adventitious bud is differentiated from this callus, the PI promoter is inactivated so that the LacI protein is not expressed. Therefore, expression of the R gene is accelerated by the histone H3 promoter which shows its activity in meristematic cells of buds, so that the recombinant enzyme R is produced and the cassette interposed between the Rs recognizing sequences is removed (Fig. 6(B)). In addition, the PI promoter is again activated in the floral meristematic cells obtained after elimination of the cassette, but since the LacI gene which produces LacI protein is no longer present, the Bax gene does not undergo inhibition by this LacI protein and its expression is accelerated by the action of the PI promoter to induce cell death (Fig. 6(C)).

### 1. Construction of vector pMS-Bax1-ipt for plant gene introduction

By constructing a vector of the present invention in which the ipt gene encoding a cytokinin synthase was introduced as a negative selectable marker gene into the constitutional unit B, a vector pMS-Bax1-ipt for plant gene introduction into which this was introduced (the vector has been deposited on April 6, 2004, as FERM BP-10003 in International Patent Organism Depositary, National Institute of Advanced Industrial Science and Technology (Tsukuba Central 6, 1-1, Higashi 1-chome, Tsukuba-shi, Ibaraki 305-8566, JAPAN)) was obtained.

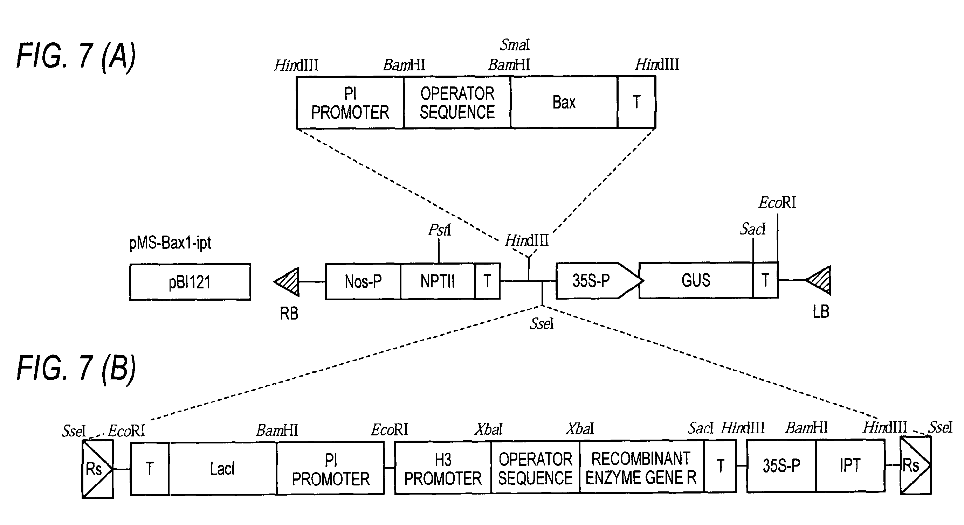

That is, as shown in Fig. 7, in this vector, a DNA fragment having a restriction enzyme *Hin*dIII recognition site in both termini and containing the ipt gene connected to the 35S promoter is inserted into the constitutional unit B of the vector pMS-Bax1 for plant gene introduction constructed in Example 1, and this vector can be effectively used for the purpose of sterilizing, by the method of the present invention, a plant individual into which a useful gene has already been introduced by using a selectable marker gene such as a kanamycin resistance gene.

### 2. Introduction of pMS-Bax1-ipt into tobacco plant

A transformant was obtained by introducing the above-described pMS-Bax1-ipt into a tobacco plant (*Nicotiana tabacum* SR-1) A2 (Kawaoka *et al., Plant J.,* 22: 289-301 (2000)) into which an antisense cDNA of the Ntlim1 gene connected to the 3 5 S promoter has already been introduced together with a kanamycin resistance gene as the selectable marker gene, in the same manner as in Example 1-1. However, since the ipt gene is inserted into this pMS-Bax1-ipt, in the case of a plant tissue into which this vector has been introduced, a plant hormone is not necessary in carrying out redifferentiation of an adventitious bud. Therefore, also in this Example, a hormone-free medium (MS inorganic salts, 3% sucrose, 0.25% gellan gum, 500 mg/l carbenicillin) was used as the medium for shoot formation. In addition, since the adventitious bud obtained in this case also showed a form of a multiple bud due to the influence of ipt gene, the foliage elongated from this multiple bud was cut off and used as the material of rooting.

### 3. Morphology of flower bud tissue

When morphology of flower buds of the transformant obtained in the above-described manner was observed with the naked eye, it was found that the flower bud tissue was died out in 4 individuals among 10 individuals Based on this result, it was confirmed that the vector of the present invention can also effectively introduce the sterile character into the transformant already prepared using kanamycin as the selectable marker gene.

While the present invention has been described in detail and with reference to specific embodiments thereof, it will be apparent to one of skill in the art that various changes and modifications can be made therein without departing from the spirit and scope thereof.

This application is based on Japanese patent application No. 2003-135141 filed on April 7, 2003, the entire contents of which are incorporated hereinto by reference. All references cited herein are incorporated in their entirety.

### Industrial Applicability

The present invention provides a vector in which a structural gene capable of exerting effect upon the phenotype of plants is positioned as the desired gene under the control of a regulatory factor which acts in a cascade-like manner. According to the present invention, expression of a desired gene can be properly controlled, by introducing the desired gene into a plant cell using such a vector and preparing a recombinant plant by culturing the plant cell according to the culturing stage or according to the cultured tissue or organ.

That is, according to the above-described vector provided by the present invention, expression of the desired gene in a callus can be inhibited without bringing a failure in producing a recombinant plant.

Particularly, the present invention has a great effect on production of sterile plants.

### Free Text of Sequence Listing

SEQ ID NO:1-Explanation of synthetic sequence: synthetic DNA
SEQ ID NO:2-Explanation of synthetic sequence: synthetic DNA
SEQ ID NO:3-Explanation of synthetic sequence: synthetic DNA
SEQ ID NO:4-Explanation of synthetic sequence: synthetic DNA
SEQ ID NO:5-Explanation of synthetic sequence: synthetic DNA
SEQ ID NO:6-Explanation of synthetic sequence: synthetic DNA

## Claims

1. A vector which comprises the following constitutional units A and B positioned on the same DNA molecule or on different DNA molecules, respectively, said vector comprising:
a promoter, as Promoter 1, which shows its activity in at least a callus and a plant tissue where a desired gene should be expressed,
a promoter, as Promoter 2, which shows its activity in at least a callus, and
a promoter, as Promoter 3, which shows its activity in at least a plant tissue where Promoter 1 and Promoter 2 do not show their activities,
A: a DNA sequence comprising Promoter 1, an expression inhibitory sequence and a desired gene in which expression is accelerated by Promoter 1 and expression is inhibited by the expression inhibitory sequence;
B: a DNA sequence comprising Promoter 2, an expression inhibitory gene in which expression is accelerated by Promoter 2 to exert function of expression inhibition by the expression inhibitory sequence, Promoter 3, the expression inhibitory sequence, and a gene of a removal reaction-catalyzing enzyme in which expression is accelerated by Promoter 3 and expression is inhibited by the expression inhibitory sequence, wherein the DNA sequence is removed by expression of the gene of a removal reaction-catalyzing enzyme.

2. The vector according to claim 1, wherein
Promoter 1 is a promoter which shows its activity in at least a floral meristematic cell and a callus, but does not show its activity in a meristematic cell of a bud;
Promoter 2 is a promoter which shows its activity in at least a callus, but does not show its activity in a meristematic cell of a bud, Promoter 3 is a promoter which shows its activity in at least a meristematic cell of a bud, and
the desired gene is a cell function inhibitory gene.

3. The vector according to claim 1 or 2, wherein Promoter 1 and Promoter 2 each is a promoter which regulates expression of PISTILLATA (PI) gene, APETALA1 (AP1) gene, APETALA2 (AP2) gene, APETALA3 (AP3) gene, AGAMOUS (AG) gene, LEAFY (LFY) gene and/or SEPALLATA3 (SEP3) gene of *Arabidopsis thaliana,* or a tobacco TA29 promoter.

4. The vector according to any one of claims 1 to 3, wherein Promoter 3 is a histone H3 promoter or a histone H4 promoter of a plant, or a promoter which regulates expression of SHOOT MERISTEMLESS (STM) gene and/or CUP-SHAPED COTYLEDOM (CUC) gene of *Arabidopsis thaliana.*

5. The vector according to any one of claims 1 to 4, wherein the desired gene is a gene encoding a cytotoxin.

6. The vector according to claim 5, wherein the gene encoding a cytotoxin is a gene encoding Bax, RNase, a protease or a DAM methylase.

7. The vector according to any one of claims 1 to 6, wherein the expression inhibitory sequence is an operator sequence and the expression inhibitory gene is a gene encoding an operator sequence-binding protein.

8. The vector according to claim 7, wherein the operator sequence is a sequence in which a transcription activation region is deleted from lacI gene or yeast GAL4 gene.

9. The vector according to any one of claims 1 to 8, wherein the gene of a removal reaction-catalyzing enzyme is a recombinant enzyme gene in a site-specific recombination system, and the constitutional unit B is interposed between two recognizing sequences which are recognized by a recombinant enzyme encoded by the recombinant enzyme gene and mutually face the same direction.

10. A method for producing a plant transformant while inhibiting expression of a desired gene in a callus, which comprises introducing a gene into a plant cell using the vector according to claim 1, and culturing the plant cell to redifferentiate a plant tissue or organ via a callus.

11. A method for producing a sterile plant, which comprises introducing a gene into a plant cell using the vector according to any one of claims 2 to 9, culturing the plant cell to propagate a callus, and redifferentiating a bud from the callus to produce a plant individual.


SEQUENCE LISTING

<110> Nippon Paper Industries, Co., Ltd.

<120> Vector for introducing a gene into a plant and method for producing
transgenic plants using the vector

<130> P04818200

<150> JP 2003-135141
<151> 2003-04-07

<160> 6

<170> PatentIn version 3.1

<210> 1
<211> 29
<212> DNA
<213> Artificial Sequence

<220>
<223> Description of artificial sequence: primer for PCR

<400> 1
aggccccggg gggagcggcg gtgatggcg 29

<210> 2
<211> 30
<212> DNA
<213> Artificial Sequence

<220>
<223> Description of artificial sequence: primer for PCR

<400> 2
cagagctctg ccataattta tggaggaaaa 30

<210> 3
<211> 45
<212> DNA
<213> Artificial Sequence

<220>
<223> Description of artificial sequence: operator

<400> 3
gatccattgt gagcgctcac aatacgtatt gtgagcgctc acaat                45

<210> 4
<211> 45
<212> DNA
<213> Artificial Sequence

<220>
<223> Description of artificial sequence: operator

<400> 4
gatcattgtg agcgctcaca atacgtattg tgagcgctca caatt                45

<210> 5
<211> 45
<212> DNA
<213> Artificial Sequence

<220>
<223> Description of artificial sequence: operator

<400> 5
ctagaattgt gagcgctcac aatacgtatt gtgagcgctc acaat                45

<210> 6
<211> 45
<212> DNA
<213> Artificial Sequence

<220>
<223> Description of artificial sequence: operator

<400> 6
ctagattgtg agcgctcaca atacgtattg tgagcgctca caatt                45